# EUROPEAN PATENT SPECIFICATION

(11) **EP 0 638 640 B1**
(45) Date of publication and mention of the grant of the patent: **22.03.2000**
(21) Application number: 93121101.5
(22) Date of filing: 30.12.1993
(51) Int. Cl.: C12M 1/20, B01L 3/00, C12M 3/06, C12M 1/12

(54) **Culture vessel**
Kulturgefäss
Récipient de culture

(30) Priority: 12.08.1993 US 105830
(43) Date of publication of application: 15.02.1995
(73) Proprietor: Becton, Dickinson and Company, Franklin Lakes, New Jersey 07417-1880 (US)
(72) Inventor: Stevens, Timothy A., Madison, New Jersey 07940 (US); Mussi, Edward F., Hewitt, New Jersey 07421 (US); Henderson, Douglas P., Morristown, New Jersey 07960 (US); Tyndorf, Tadeusz A., Manalapan Township, New Jersey 07726 (US)
(74) Representative: Selting, Günther, Dipl.-Ing.

(56) References cited:
- EP-A- 0 183 973
- EP-A- 0 239 697
- EP-A- 0 495 213
- EP-A- 0 590 485
- EP-A- 0 590 513
- WO-A-92/07063
- US-A- 4 871 674
- Webster's encyclopedic unabridged dictionary, 1994, p. 592

## Description

This invention relates to an apparatus useful for procedures in growing cells or tissue culture in vitro and more particularly for supporting, positioning and/or centering cell culture inserts that are used in the procedures.

Culture vessels are described in U.S. Patent Nos. 4,495,289 and 5,026,649 and European Patent Application No. 0 483 620 A2. Culture vessels comprise wells which generally have a circular shape and size which permits the introduction therein of a cell culture insert having a membrane upon which cell attachment, growth and differentiation occur. The culture vessels containing the wells are typically rectangular and have a standard size in order to accommodate standard analytical apparatus.

Conventional cell culture inserts used in culture vessels are described in U.S. Patent Nos. 4,871,674 and 5,026,649. U.S. Patent No. 4,871,674 discloses a cell culture insert which comprises discontinuous projecting parts for hanging the cell culture insert on an upper circumferential part of the culture vessel. U.S. Patent No. 5,026,649 discloses a cell culture insert which comprises a projecting part for hanging the culture cell on an upper circumferential part of the culture vessel, and further includes openings in the sidewalls for exchanging gas and for pipeting sample.

EP-A-0 239 697 discloses an apparatus for growing tissue cultures in vitro, said apparatus comprising a culture vessel and a cell culture insert suspended in a well of the vessel. The insert comprises a circular flange resting on a supporting surface of the vessel. The insert comprises a permeable membrane forming the bottom surface thereof. The insert may be rotated with respect to the vessel. The insert comprises a cutout for introducing a pipette to the wall of the insert into the culture vessel.

Although there are a number of culture vessels and cell culture inserts commercially available and described in patent publications, it is believed that there are no culture vessels or cell culture inserts available that can be used together to support, position and/or center a cell culture insert without interfering with the growing of tissue cultures in vitro.

It is an object of the invention to provide an apparatus for use in growing tissue cultures in vitro which allows for positioning cell culture inserts in such a way that contamination is reduced.

The apparatus of the present invention is defined by claim 1.

The apparatus preferably comprises a test plate including an upper surface, a lower surface, and a plurality of wells. Each well is substantially disposed between the upper and lower surface of the test plate. Each well comprises a sidewall, a bottom surface and a raised mouth surface. Most preferably, the raised mouth surface of each well includes a means for supporting a cell culture insert. This means is desirably raised from the upper surface.

The raised mouth surface preferably includes means for positioning and/or centering a cell culture insert. The preferred means is at least one integral lip portion extending from the raised mouth surface. The integral lip portion may be arranged to assist in positioning and/or centering a cell culture insert without limiting the movement of the cell culture insert.

Desirably, the cell culture insert that may be used with the device comprises an upper portion, a lower portion and sidewalls extending from the upper portion to the lower portion. The lower portion includes a bottom surface which may have attached thereto a flat permeable membrane. The upper portion includes a top surface which carries at least one outwardly extending flange.

The flange allows the cell culture insert to be supported on the top surface of the well of the device so that there is clearance between the bottom of the membrane and the bottom surface of the well. The clearance provides a controlled static head and diffusion for the fluid in the device so that cells can be properly cultured.

The cell culture insert may further include at least one support footing on the bottom surface. The footing may provide a degree of clearance between the membrane and the bottom surface of the well.

Most preferably, the outer surface of the sidewall of the cell culture insert may further include means for restricting and/or preventing the flange of the cell culture insert from falling into the well of the device.

Although the apparatus of the present invention may be configured in a circle with one well, the preferable form of the device is a multi-sided structure with wells in an ordered array of parallel rows. Furthermore, the integral lip portions on the raised mouth surface of each well may be positioned so that the integral lip portion of one well does not face an integral lip portion of an adjacent well. This positioning of the integral lip portions assures that the flanges of adjacent cell culture inserts positioned in the wells can be accessible, do not contact one another and that the cell culture insert may be aseptically placed and removed into or out of the well of the device.

The apparatus may further include a removable lid which can be positioned over the upper surface. The lid includes a top wall and a peripheral skirt that extends from the top wall. The lid serves to prevent the loss of sample from the well interior, to protect the contents of the apparatus from the environment and to protect the user from the contents of the apparatus should it contain a harmful or potentially harmful material.

The apparatus preferably receives a cell culture insert in the well. The cell culture insert is positioned into the well wherein the flanges of the cell culture insert are supported on the raised mouth surface of the well and located between the integral lip portions. The integral lip portions assist in substantially positioning and/or centering the cell culture insert in the well and the raised mouth surface of the well substantially restricts the cell culture insert from falling into the well. Movement of the cell culture insert within the well may be such that the cell culture insert wall and the well sidewall can touch at one or more locations.

Alternatively, the upper mouth surface of the well may comprise at least one integral standing rib portion and the flange of the cell culture insert may comprise a slot to engage the standing rib portion. In this embodiment, the cell culture insert is positioned in the well wherein the slot of the flange engages the integral standing rib portion of the well. The rib portion assists in substantially positioning and/or centering the cell culture insert in the well while movement of the cell culture insert in the well may be such the cell culture insert wall and the side wall of the well can touch at one or more locations.

The exterior dimensions of the portion of the cell culture insert within the well of the device are sufficiently less than the well diameter to allow a pipet or similar device to be positioned in the space between the well of the device and the cell culture insert for fluid filling or aspiration without disturbing or removing the cell culture insert from the well of the device. The space allows the pipet to reach the bottom of the well and introduce or remove medium from beneath the membrane and about the outer surface of the sidewall of the cell culture insert without contaminating the upper surface of the membrane.

The device and related removable lid may be formed in different sizes and geometric configurations so as to be used with different size and geometric configured cell culture inserts. The removable lid may be formed to be positioned over the upper surface of the device in one orientation so as to reduce cross contamination between the wells in the event the lid is repositioned over the upper surface of the device. The device and the removable lid are preferably made of an optically clear plastic to facilitate viewing of the wells and cell culture inserts.

An important feature of the present invention is that in a multi-well configuration, flange to flange contact between the cell culture inserts is prevented so as to reduce contamination. This is accomplished due to the orientation of the integral lip portions on each well wherein the integral lip portion of one well does not face or interfere with an integral lip portion of an adjacent well.

A further feature of the present invention is that the top surface configuration of the well of the device may facilitate the aseptic placement and removal of the cell culture insert into or out of the well of the device with tweezers or forceps, as well as providing support to the cell culture insert when placed in the well of the device.

### DESCRIPTION OF THE DRAWINGS

FIG. 1 is an exploded perspective view of a multi-well culture vessel and a cell culture insert.
FIG. 2 is a cross sectional view taken along lines 2-2 of FIG. 1 illustrating the means available for supporting, positioning and/or centering a cell culture insert in the well of a culture vessel.
FIG. 3 is an exploded perspective view of a multi-well culture vessel and the optional removable lid not attached.
FIG. 4 is a top view of FIG. 1 illustrating the cell culture insert supported in the well of a culture vessel.
FIG. 5 is a cross-sectional view taken along lines 5-5 of FIG. 4 illustrating where a pipet tip, shown in phantom, may enter the space between the cell culture insert and the well of the culture vessel.
FIG. 6 is an exploded perspective view of an alternate embodiment of the present invention, a one-well culture vessel.
FIG. 7 is an exploded perspective view of a multi-well culture vessel and a cell culture insert similar to FIG. 1 illustrating an additional embodiment of the invention.
FIG. 8 is an exploded perspective view of the multi-well culture vessel of FIG. 7 and the optional removal lid not attached.
FIG. 9 is a top view of FIG. 7 illustrating the cell culture insert supported in the well of a culture vessel.
FIG. 10 is a cross-sectional view taken along lines 10-10 of FIG. 9 illustrating where a pipet tip, shown in phantom, may enter the space between the cell culture insert and the well of the culture vessel.
FIG. 11 is an exploded perspective view of a one-well culture vessel similar to FIG. 7, illustrating an additional embodiment of the invention.

### DETAILED DESCRIPTION

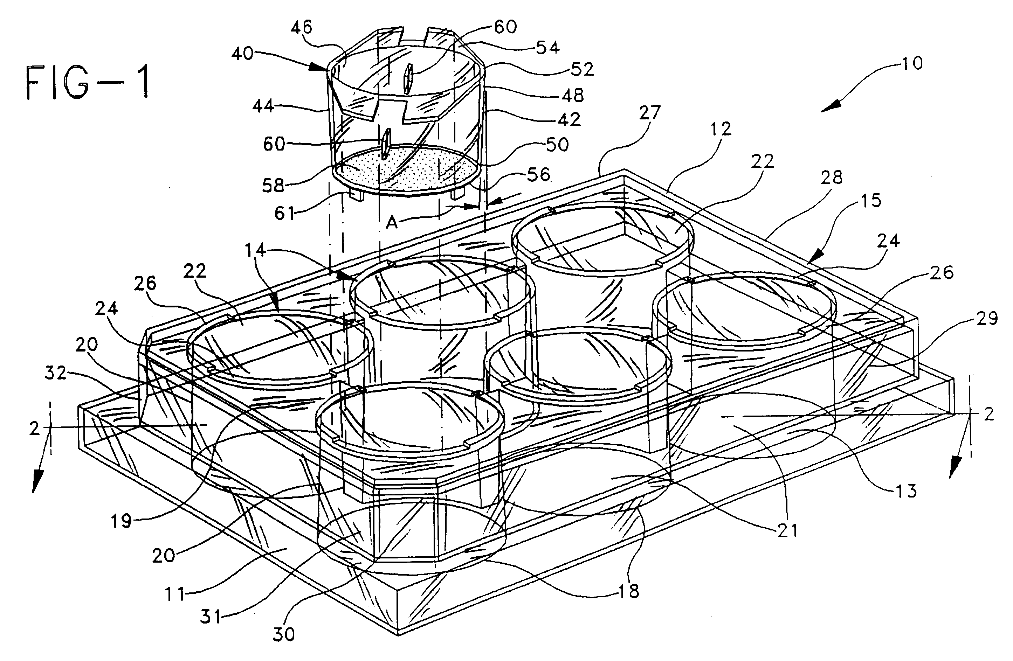

An apparatus **10** for growing tissue cultures as shown in FIG. 1 includes a culture vessel **15** and a cell culture insert **40**. Although a six well culture vessel is shown, it should be appreciated that the culture vessel may have one, eight, twelve, twenty-four or some other number of wells selected for the particular purpose for which the apparatus system is used.

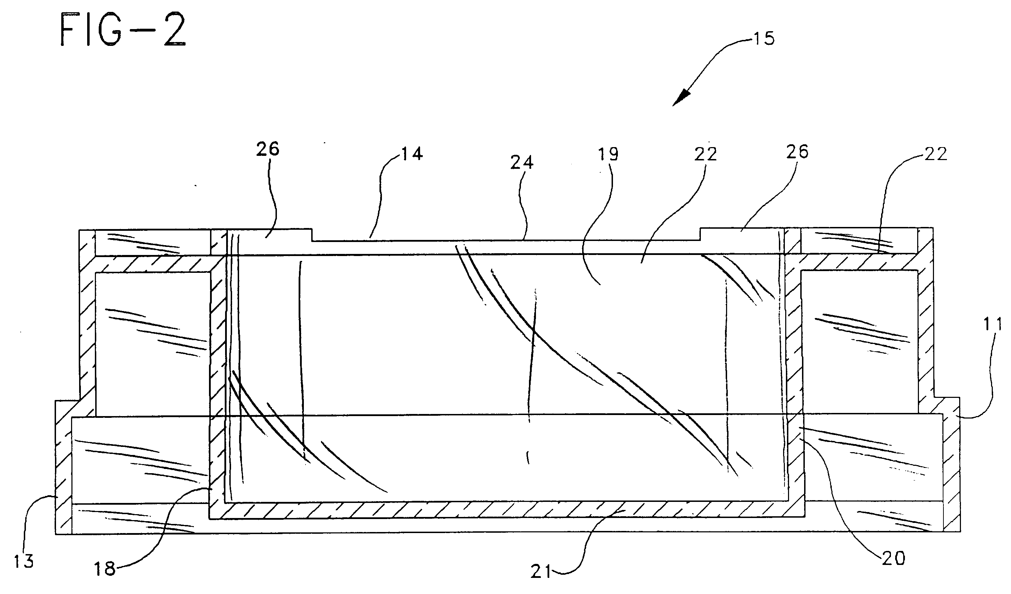

As shown in FIGS. 1 and 2, culture vessel **15** includes a base **11** with an upper surface **12** and lower surface **13**. The culture vessel further includes a number of wells **14** each comprising a sidewall **20** extending from the upper surface to the lower surface. A top portion **19** of the well comprises an open end **22**, a top surface **24** extending from the upper surface and two integral lip portions **26** extending from the top surface. A bottom portion **18** of the well comprises a closed end **21**.

Base **11**, as illustrated in FIG. 1, typically is transparent and may be molded, for example, of polyvinylchloride or polystyrene. The culture vessel further includes sidewalls **27**, **28**, **29**, **30**, **31** and **32** that extend from upper surface **12** to lower surface **13**. Sidewalls **27** and **29**, and **28** and **31** are substantially parallel to each other respectively. Sidewall **30** is located between sidewalls **29** and **31** and sidewall **32** is located between sidewalls **31** and **27**.

As shown in FIG. 1, the cell culture insert includes a body wall **42** having an outer wall surface **44** and an inner wall surface **46**. Body wall **42** extends from an upper portion **48** to a lower portion **50**. The body wall may taper from the upper portion to the lower portion. Although it is within the purview of this invention that draft angle **A** of the cell insert be from about 5 degrees to about 8 degrees, it is most preferred that the draft angle is 6 degrees. Upper portion **48** comprises a top surface **52** which carries two outwardly extending flanges **54**. The flanges are located opposite from one another approximately 180° apart and are discontinuous. Flanges **54** comprise a horizontal orientation for resting on top surface **24** of well **14**.

Lower portion **50** comprises a bottom surface **56** to which a microporous membrane **58** is adhered. The membrane may be made of suitable material including, but not limited to, perforated inert film, hydrated gel, or a layered combination.

In the embodiment illustrated, two projecting or extending tabs **60** are located on outer wall surface **44** of the cell culture insert. The tabs are located opposite from one another or approximately 180° apart and approximately half the distance between the upper and lower portions. The projecting tabs provide stability to the cell culture insert when it is in the well and shifted for pipette insertion. In the event the cell culture insert is shifted and one of the flanges falls into the well, the projecting tabs will substantially prevent the opposite flange from falling into the well.

The cell culture insert may further include feet or supports **61** located on the bottom surface. The feet are located opposite from one another or approximately 180° apart. Feet **61** are used when the extending flanges do not provide adequate support to the cell culture insert in a particular well configuration. It is most desirable that a space be maintained between the membrane on the bottom surface of the cell culture insert and the well of the culture vessel so that cells may be cultured on both sides of the membrane. Feet **61** further provide support to the cell culture insert when placed on a flat surface.

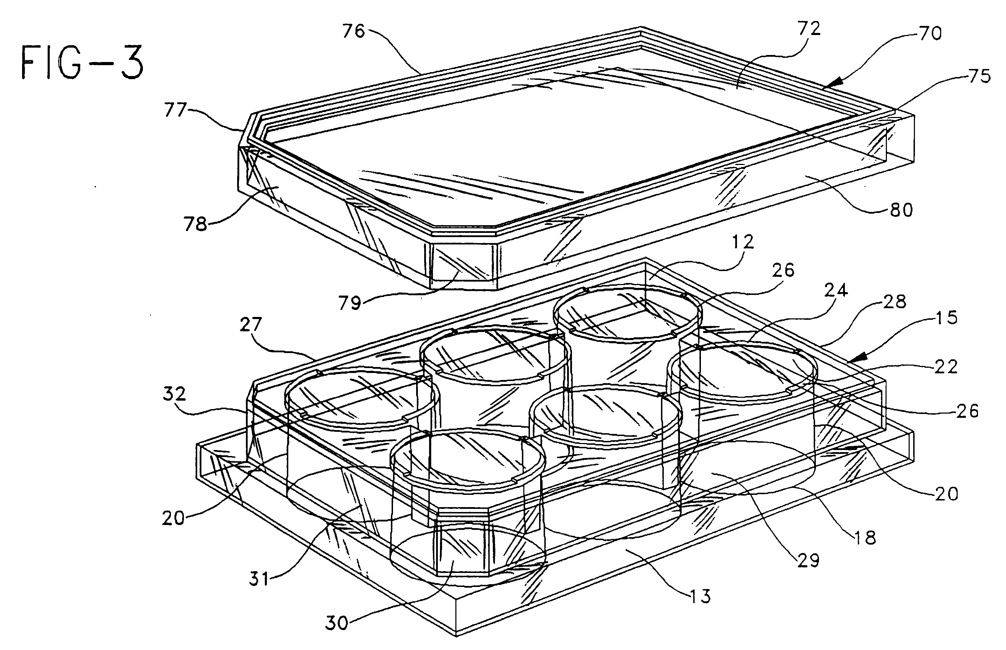

As shown in FIG. 3, the apparatus further includes a removable lid **70**. Removable lid **70** includes a top wall **72**, and peripheral sides **75**, **76**, **77**, **78**, **79** and **80** that extend from the top wall. The lid removably covers the upper surface of the culture vessel wherein the peripheral sides abut closely with the base of the culture vessel. In particular peripheral sides **77** and **79** of the lid abut with sidewalls **30** and **32** of the culture vessel. The lid is configured so that peripheral sides **77** and **79** are unable to abut with sidewalls **27**, **28**, **29** and **31** of the culture vessel. Therefore the lid may only be mated with the culture vessel in one way so that cross contamination is minimal between the wells of the culture vessel.

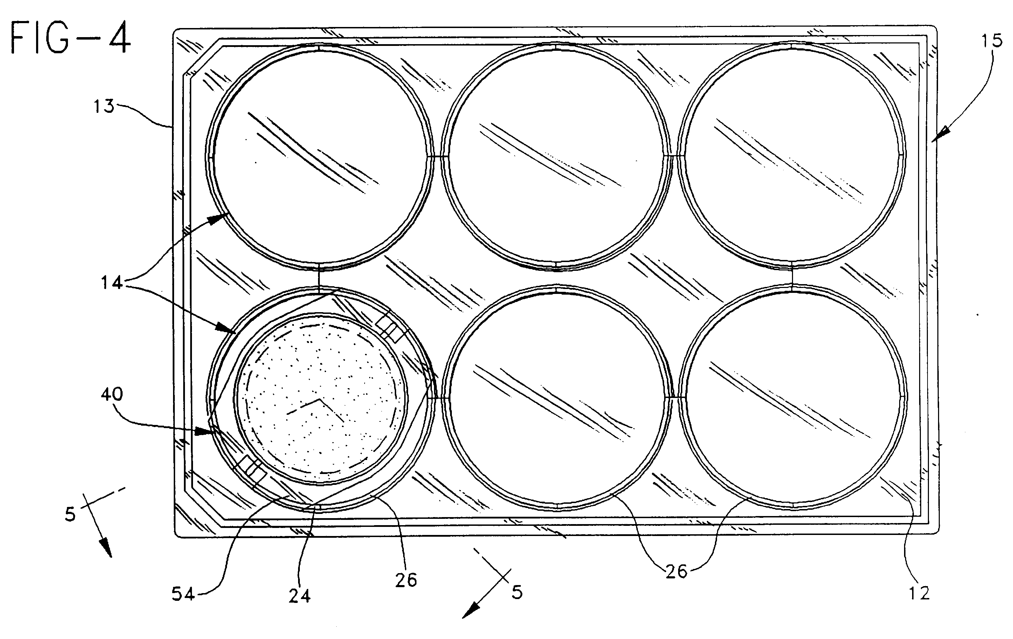

As shown in FIG. 4, wells **14** are sufficiently spaced from one another and the integral lip portions are oriented so as not to face or interfere with the integral lip portions of adjacent wells. The integral lip portions of each well are located opposite from one another or approximately 180° apart. This particular orientation of the integral lip portions substantially prevents flange to flange contact between the cell culture inserts and substantially reduces cross contamination between the cell culture inserts.

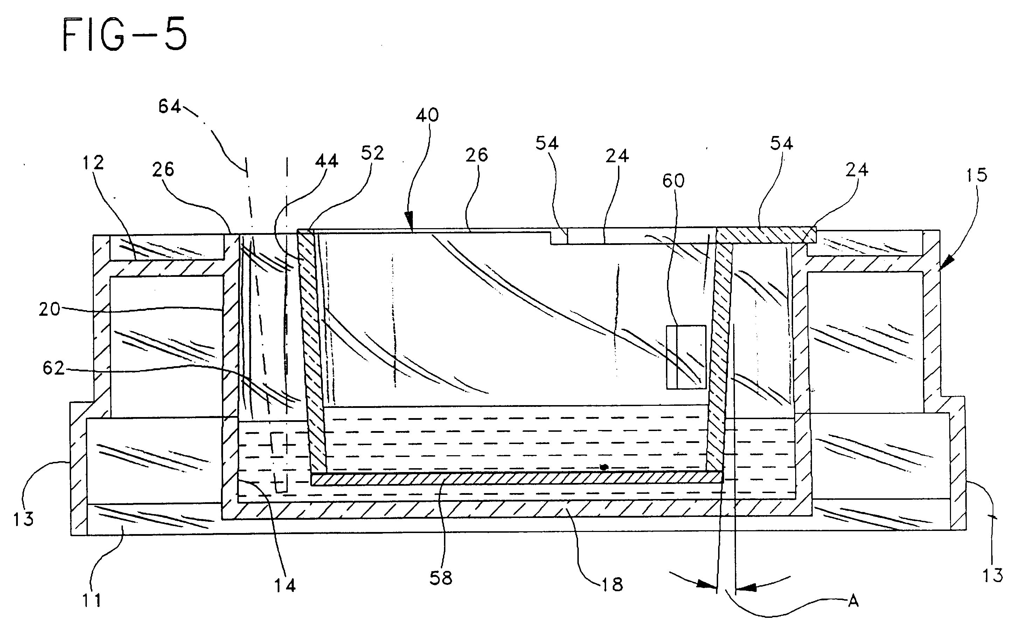

FIG. 5 illustrates the orientation of the cell culture insert as supported in culture vessel **15** wherein flange **54** is positioned on top surface **24** and between integral lip portions **26**. Top surface **24** supports the cell culture insert and integral lip portions **26** assist in positioning and/or centering the cell culture insert without limiting movement of the cell culture insert. FIG. 5 also illustrates a pipet tip **64**, in phantom, which enters the space **62** between outer wall surface **44** of the cell culture insert and sidewall **20** of the culture vessel.

The centering feature provided by integral lip portions **26** can be seen in Fig. 5. Cell culture insert **40** is set within well **14** and spaced sufficiently from well sidewall **20** as facilitated by top surface **24** and integral lip portions **26**. Thus, capillary action should not occur to cause solution or media in space **62** from wicking up outer surface **44** and entering the interior of the cell culture insert or spilling from well **14**. The movement of the cell culture insert, however, may be such that the cell culture outer wall surface and the sidewall of the well can touch at one or more locations.

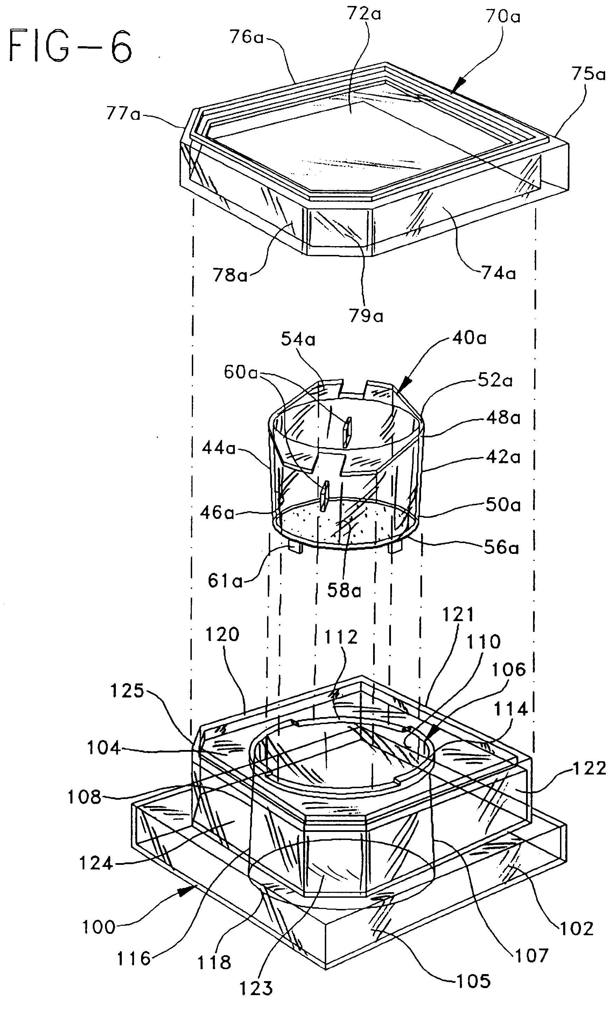

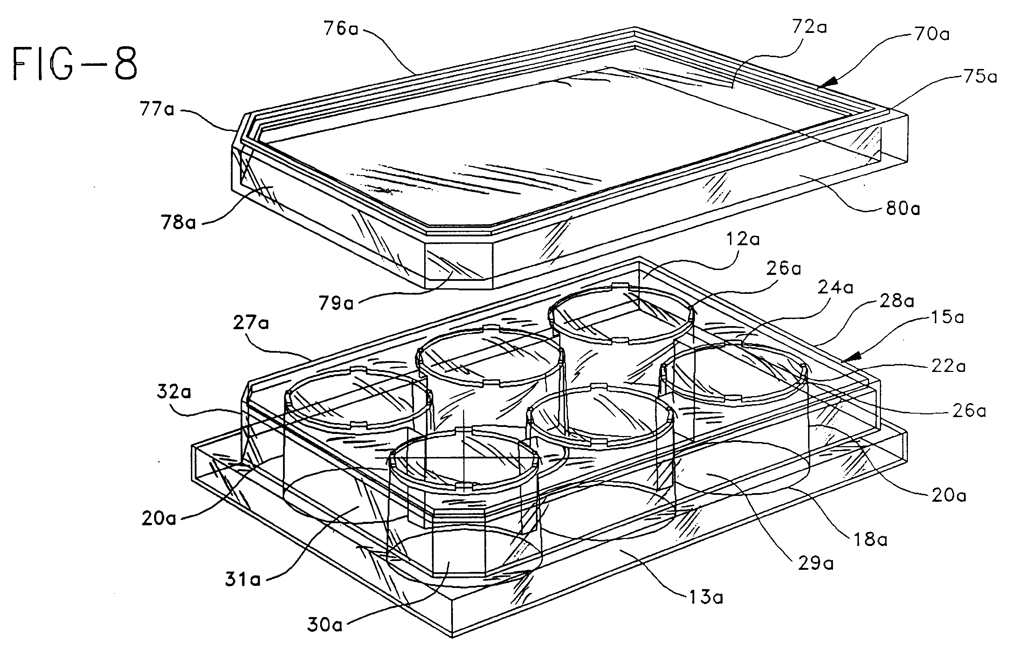

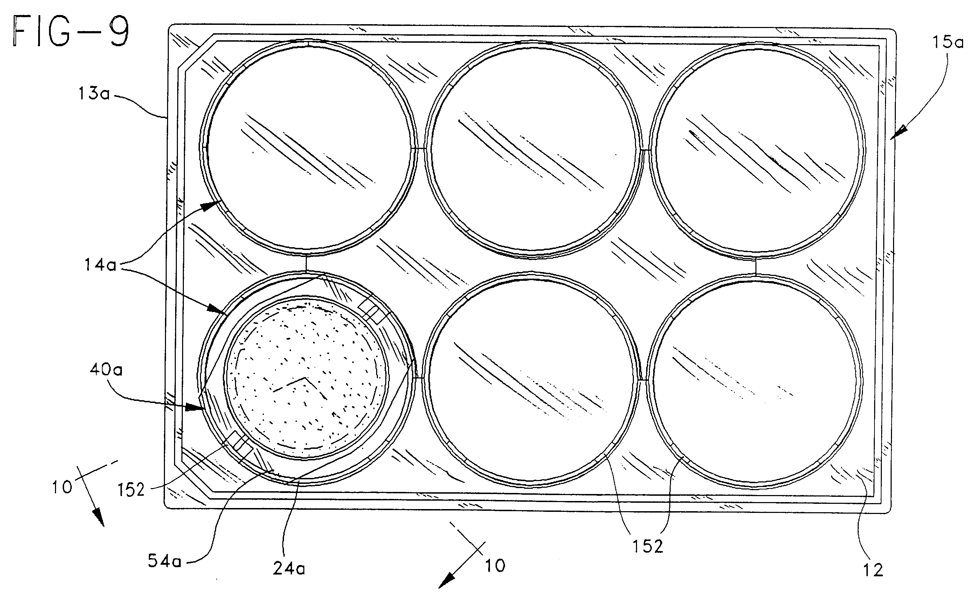

The invention, as shown in FIGS. 6-11 includes many components which are substantially identical to the components of FIGS. 1-5. Accordingly, similar components performing similar functions will be numbered identically to those components of FIGS. 1-5, except that a suffix "a" will be used to identify those similar components in FIG. 6-11.

As illustrated in FIG. 6, a further embodiment of the invention includes a one well apparatus **90** having a culture vessel **100**, a cell culture insert **40a** and a removable lid **70a**. The culture vessel includes a base **102** with an upper surface **104** and a lower surface **105**. A well **106** comprising a sidewall **107** extends from the upper surface to the lower surface. A top portion **108** of the well comprises an open end **110**, a top surface **112** extending from the upper surface and two integral lip portions **114** extending from the top surface. A lower portion **106** of the well comprises a closed end **118**.

Base **102** typically is transparent and may be molded, for example, of polyethylene terephthalate. The culture vessel further includes sidewalls **120**, **121**, **122**, **123**, **124** and **125** that extend from upper surface **104** to lower surface **105**.

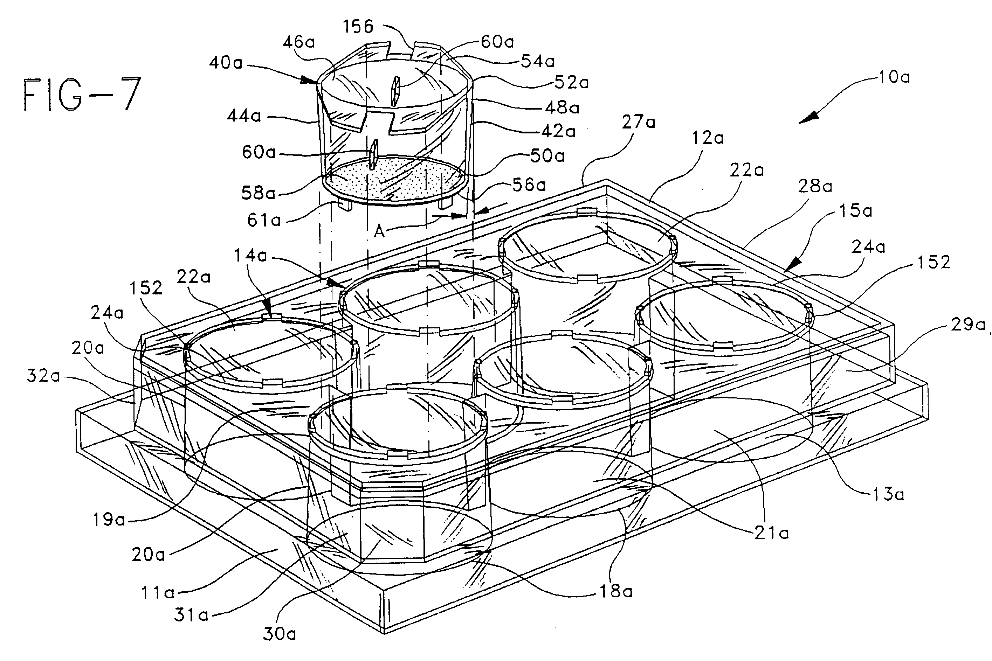

As illustrated in FIGS. 7-9, a further embodiment of the invention includes a culture vessel **15a** wherein the top portion **19a** of the well comprises two integral standing ribs portions **152** extending from top surface **24a** and a modified cell culture insert **40a** wherein each flange has a slot **156** to engage standing rib portions **152**. The flanges are located opposite from one another approximately 180° apart and have a horizontal orientation for resting on top surface **24a** of the well. Although it is within the purview of this invention that body wall **42a** of cell culture insert **40a** extends from upper portion **48a** to lower portion **50a**, it is most preferred that body wall **42a** tapers from upper portion **48a** to lower portion **50a**. Although it is within the purview of this invention that draft angle **A** of the cell insert be from about 5 degrees to about 8 degrees, it is most preferred that the draft angle is 6 degrees.

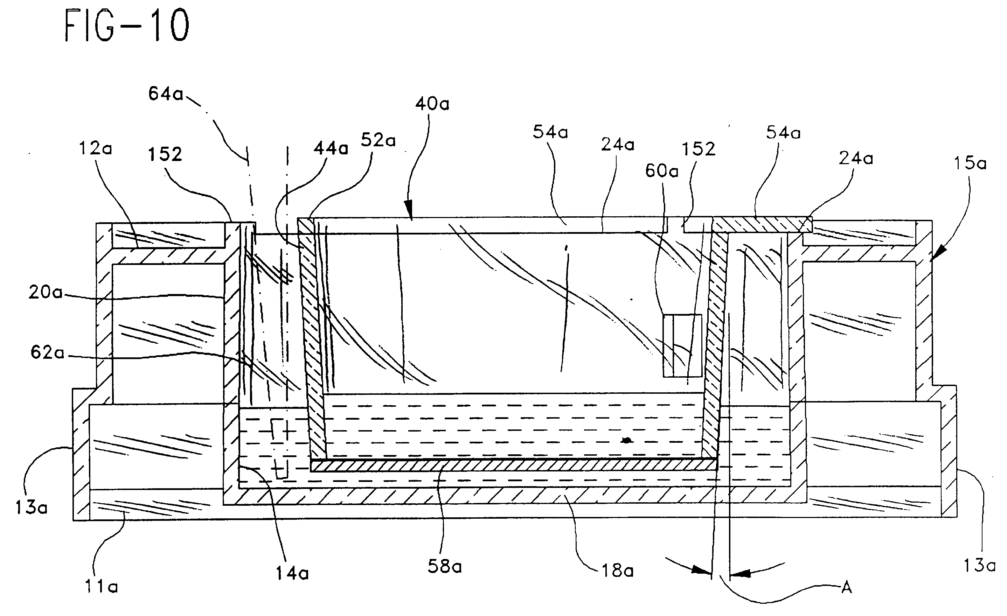

FIG. 10 illustrates the orientation of the cell culture insert as supported in culture vessel **15a** wherein slot **156** of the flange engages integral standing rib **152**. Top surface **24a** supports the cell culture insert and the integral standing ribs assist in positioning and/or centering the cell culture insert.

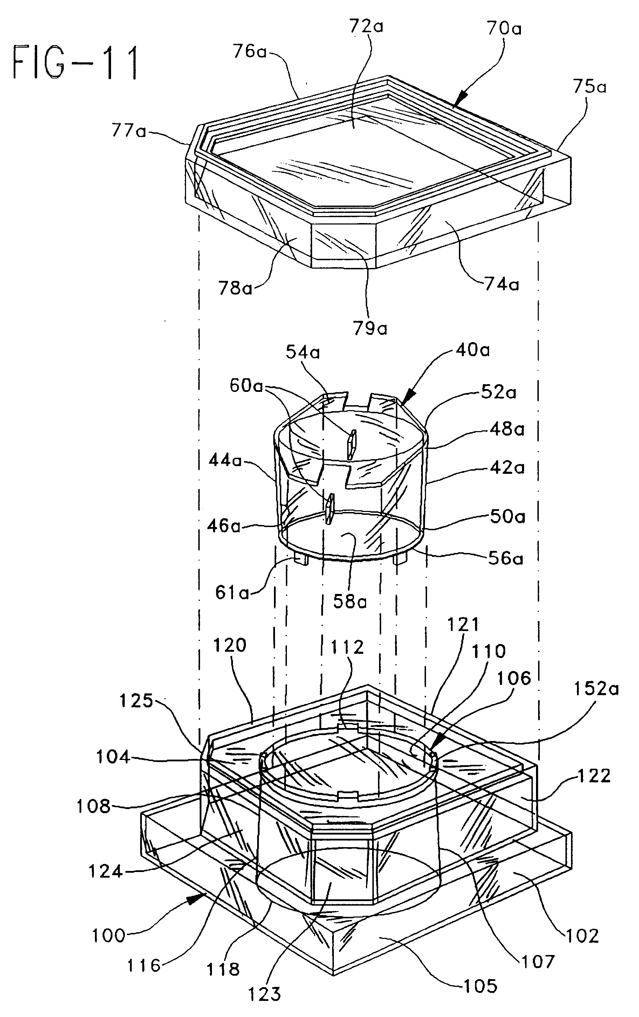

FIG. 11 illustrates an additional embodiment of the invention, a one well apparatus.

As practitioners-in-the-art will understand, the culture vessel and cell culture insert of the present invention may be comprised of simple moldable parts which may be mass produced from a variety of materials, including, for example, polyethylene, polystyrene, polyethylene terephthalate, and polypropylene. As will be understood further by practitioners in the art, materials should be selected which provide a small degree of resiliency for the purpose of providing ease of insertion of the cell culture inserts into the culture vessel and ease of use for subsequent examination of the developed cultured cells.

## Claims

1. Apparatus for use in growing tissue cultures in vitro comprising:
a culture vessel (15;15a;100) comprising an upper surface (12;12a;104), a lower surface (13;13a;105), a plurality of wells (14;14a;106) extending from said upper surface (12;12a;104) to said lower surface (13;13a;105), a raised mouth surface (24;24a) extending from each said upper surface of each well, and
a cell culture insert (40;40a) suspended in one of said wells (14;14a;106) and comprising an upper portion (48;48a), a lower portion (50;50a), a sidewall (42;42a) comprising an inner (46;46a) and an outer surface (44;44a) and extending from said upper portion (48;48a) to said lower portion (50;50a), and a permeable membrane (58;58a) attached to said lower portion and a flange (54;54a),
**characterized in that**
the cell culture vessel (15;15a;100) has at least one projecting portion (152;152a) extending from said raised mouth surface (24;24a), said projecting portion enganging with a slot (156) in the flange (54;54a) of the cell culture insert (40;100).

2. The apparatus of claim 1 wherein said cell culture insert (40;40a) comprises two projecting portions (152) located opposite from one another.

3. The apparatus of claim 1 or 2 wherein said cell culture insert (40;40a) further comprises two projecting tabs (60;60a) for restricting movement of said cell culture insert in said well (14;14a;106) of said culture vessel (15;15a;100) extending from said outer surface (44;44a) of said sidewall (42;42a).

4. The apparatus of claim 3 wherein said insert includes two projecting tabs (60;60a) located opposite one another on the outer surface (44;44a) of sidewall and between said upper portion (48;48a) and said lower portion (50;50a).

5. The apparatus of one of claims 1-4 wherein said culture vessel (15;15a;100) is substantially rectangular in shape and said wells (14;14a;106) are in an ordered array of substantially parallel rows.

6. The apparatus of claim 5 further comprising a plurality of cell culture inserts (40;40a).

7. The apparatus of one of claims 1-6 wherein said projecting portions (152) of the wells (14;14a;106) are in an ordered array to substantially prevent the flange (54;54a) of one cell culture insert (40;40a) from contacting the flange of another cell culture insert.

8. The apparatus of one of claims 1-7 further comprising a removable lid (70;70a) having a top wall (72) and a peripheral skirt that extends from said top wall.

9. The apparatus of one of claims 1-8 wherein said cell culture insert (40;40a) further comprises a draft angle (A) from about 5 degrees to about 8 degrees.

10. The apparatus of claim 9 wherein said draft angle (A) is about 6 degrees.

## Patentansprüche

1. Vorrichtung zur Verwendung beim In-Vitro-Züchten von Gewebekulturen, mit:
einem Kulturgefäß (15; 15a; 100) mit einer Oberseite (12; 12a; 104), einer Unterseite (13; 13a; 105), mehreren sich von der Oberseite (12; 12a; 104) zur Unterseite (13; 13a; 105) erstreckenden Aufnahmen (14; 14a; 106), einer erhabenen Öffnungsfläche (24; 24a), die sich von jeder Oberseite jeder Aufnahme aus erstreckt, und
einem Zellkultureinsatz (40; 40a), der in einer der Aufnahmen (14; 14a; 106) hängend gehalten ist und einen oberen Bereich (48; 48a), einen unteren Bereich (50; 50a), eine sich vom oberen Bereich (48; 48a) zum unteren Bereich (50; 50a) erstreckenden Seitenwand (42; 42a) mit einer inneren (46; 46a) und einer äußeren Fläche (44; 44a), und eine am unteren Bereich und einem Flansch (54; 54a) angebrachte durchlässige Membran (58; 58a) aufweist,
dadurch gekennzeichnet, daß
das Zellkulturgefäß (15; 15a; 100) wenigstens einen vorstehenden Bereich (152; 152a) aufweist, der sich von der erhabenen Öffnungsfläche (24; 24a) aus erstreckt, wobei der vorstehende Bereich in einen Schlitz (156) im Flansch (54; 54a) des Zellkultureinsatzes (40; 100) eingreift.

2. Vorrichtung nach Anspruch 1, bei der der Zellkultureinsatz (40; 40a) zwei einander gegenüberliegende vorstehende Bereiche (152) aufweist.

3. Vorrichtung nach Anspruch 1 oder 2, bei der der Zellkultureinsatz (40; 40a) ferner zwei vorstehende Ansätze (60; 60a) zum Begrenzen der Bewegung des Zellkultureinsatzes in der Aufnahme (14; 14a; 106) des Kulturgefäßes (15; 15a; 100) aufweist, welche sich von der äußeren Fläche (44; 44a) der Seitenwand (42; 42a) aus erstrecken.

4. Vorrichtung nach Anspruch 3, bei der der Einsatz zwei vorstehende Ansätze (60; 60a) aufweist, die einander gegenüberliegend an der äußeren Seite (44; 44a) der Seitenwand und zwischen dem oberen Bereich (48; 48a) und dem unteren Bereich (50; 50a) angeordnet sind.

5. Vorrichtung nach einem der Ansprüche 1-4, bei der das Kluturgefäß (15; 15a; 100) im wesentlichen rechteckig ist und die Aufnahmen (14; 14a; 106) in einer geordneten Anordnung von im wesentlichen parallelen Reihen vorgesehen sind.

6. Vorrichtung nach Anspruch 5, ferner mit mehreren Zellkultureinsätzen (40; 40a).

7. Vorrichtung nach einem der Ansprüche 1-6, bei der die vorstehenden Bereiche (152) der Aufnahmen (14; 14a; 106) in einer geordneten Anordnung vorgesehen sind, um den Flansch (54; 54a) eines Zellkultureinsatzes (40; 40a) im wesentlichen daran zu hindern, den Flansch eines anderen Zellkultureinsatzes zu berühren.

8. Vorrichtung nach einem der Ansprüche 1-7, ferner mit einem abnehmbaren Deckel (70, 70a) mit einer Oberwand (72) und einer Umfangsschürze, die sich von der Oberwand aus erstreckt.

9. Vorrichtung nach einem der Ansprüche 1-8, bei der der Zellkultureinsatz (40; 40a) ferner einen Schrägwinkel (A) von ungefähr 5° bis ungefähr 6° aufweist.

10. Vorrichtung nach Anspruch 9, bei der der Schrägwinkel (A) ungefähr 6° beträgt.

## Revendications

1. Dispositif destiné à être utilisé pour la croissance de cultures tissulaires in vitro, comportant :
un récipient de culture (15 ; 15a ; 100) comportant une surface supérieure (12 ; 12a ; 104), une surface inférieure (13 ; 13a ; 105), une pluralité de puits (14 ; 14a ; 106) s'étendant depuis ladite surface supérieure (12 ; 12a ; 104) jusqu'à ladite surface inférieure (13 ; 13a ; 105), une surface d'ouverture surélevée (24 ; 24a) s'étendant depuis chaque surface supérieure de chaque puits, et
une pièce rapportée de culture cellulaire (40 ; 40a) suspendue dans un desdits puits (14 ; 14a ; 106) et comportant une partie supérieure (48 ; 48a), une partie inférieure (50 ; 50a), une paroi latérale (42 ; 42a) comportant une surface intérieure (46 ; 46a) et une surface extérieure (44 ; 44a) et s'étendant depuis ladite partie supérieure (48 ; 48a) jusqu'à ladite partie inférieure (50 ; 50a), et une membrane perméable (58 ; 58a) fixée à ladite partie inférieure et un rebord (54 ; 54a),
caractérisé en ce que
le récipient de culture cellulaire (15 ; 15a ; 100) a au moins une partie en saillie (152 ; 152a) s'étendent depuis ladite surface d'ouverture surélevée (24 ; 24a), ladite partie en saillie venant en prise avec une fente (156) dans le rebord (54 ; 54a) de la pièce rapportée de culture cellulaire (40 ; 100).

2. Dispositif selon la revendication 1, dans lequel ladite pièce rapportée de culture cellulaire (40 ; 40a) comporte deux parties en saillie (152) situées à l'opposé l'une de l'autre.

3. Dispositif selon la revendication 1 ou 2, dans lequel ladite pièce rapportée de culture cellulaire (40 ; 40a) comporte de plus deux pattes en saillie (60 ; 60a) pour limiter le mouvement de ladite pièce rapportée de culture cellulaire dans ledit puits (14 ; 14a ; 106) dudit récipient de culture (15 ; 15a ; 100) s'étendent depuis ladite surface extérieure (44 ; 44a) de ladite paroi latérale (42 ; 42a).

4. Dispositif selon la revendication 3, dans lequel ladite pièce rapportée comporte deux pattes en saillie (60 ; 60a) situées à l'opposé l'une de l'autre sur le surface extérieure (44 ; 44a) de la paroi latérale et entre ladite partie supérieure (48 ; 48a) et ladite partie inférieure (50 ; 50a).

5. Dispositif selon l'une quelconque des revendications 1 à 4, dans lequel ledit récipient de culture (15 ; 15a ; 100) a une forme pratiquement rectangulaire et lesdits puits (14 ; 14a ; 106) sont disposés en matrice ordonnée ayant des rangées pratiquement parallèles.

6. Dispositif selon la revendication 5, comportant de plus une pluralité de pièces rapportées de culture cellulaire (40 ; 40a).

7. Dispositif selon l'une quelconque des revendications 1 à 6, dans lequel lesdites parties en saillie (152) des puits (14 ; 14a ; 106) sont disposées en matrice ordonnée pour vraiment empêcher que le rebord (54 ; 54a) d'une pièce rapportée de culture cellulaire (40 ; 40a) ne comprime le rebord d'une autre pièce rapportée de culture cellulaire.

8. Dispositif selon l'une quelconque des revendications 1 à 7, comportant de plus un couvercle amovible (70, 70a) ayant me paroi supérieure (72) et une enveloppe périphérique qui s'étend depuis ladite paroi supérieure.

9. Dispositif selon l'une quelconque des revendications 1 à 8, dans lequel ladite pièce rapportée de culture cellulaire (40 ; 40a) comporte de plus un angle de tirage (A) compris entre environ 5 degrés et environ 8 degrés.

10. Dispositif selon le revendication 9, dans lequel ledit angle de tirage (A) est d'environ 6 degrés.
